# EUROPÄISCHE PATENTSCHRIFT

(11) **EP 2 676 648 B1**
(45) Veröffentlichungstag und Bekanntmachung des Hinweises auf die Patenterteilung: **23.01.2019**
(21) Anmeldenummer: 13173415.4
(22) Anmeldetag: 24.06.2013
(51) Int. Cl.: B65H 29/24, A61F 13/15, B65H 39/14

(54) **Elastik-Applikator**
Elastic applicator
Applicateur élastique

(30) Priorität: 24.06.2012 DE 102012210776
(43) Veröffentlichungstag der Anmeldung: 25.12.2013
(73) Patentinhaber: Winkler + Dünnebier GmbH, 56564 Neuwied (DE)
(72) Erfinder: Heinrich, Walter, 56567 Neuwied (DE); Sanner, Dennis, 56338 Braunbach (DE)
(74) Vertreter: Walkenhorst, Andreas

(56) Entgegenhaltungen:
- EP-A1- 1 162 162
- EP-B1- 0 869 755
- WO-A1-2004/073568
- DE-A1- 3 238 051
- DE-T2- 60 031 962
- DE-T2- 60 302 531
- US-A1- 2004 035 521

## Beschreibung

Die Erfindung bezieht sich auf einen Elastik-Applikator zum Aufbringen eines elastischen Elements auf eine Materiallage umfassend einem Scheibenpaar und jeweils einer Antriebseinheit zum Antreiben der beiden Scheiben des Scheibenpaares, wobei die Scheiben des Scheibenpaares und die Antriebseinheiten zum Betrieb mit voneinander dem Betrage nach verschiedenen Geschwindigkeiten ausgelegt sind und auf ein Verfahren zum Betreiben eines Elastik-Applikators zum Aufbringen eines elastischen Elements auf eine Materiallage.
Hygieneartikel, wie etwa Binden oder Wegwerfwindeln, verfügen in der Regel über elastische Elemente, die auf der Lagenoberfläche angeordnet sein können. Diese dienen insbesondere dazu Raffungen auszubilden, damit sich der Gegenstand dem Körper des Trägers optimal anpassen kann. Das elastische Element sollte dabei mit einer möglichst gleichmäßigen Ausdehnung auf der Lagenoberfläche angeordnet sein, da erheblich ungleichmäßige Ausdehnungen zu einem schlechten Tragekomfort aber auch zu einem Auslaufen von Körperflüssigkeiten führen können.
Aus der Druckschrift DE 603 02 531 T2 ist eine Vorrichtung zum Herstellen eines derartigen Hygieneartikels bekannt, die mit Hilfe von kreisförmig angeordneten Kissen, das elastische Element aufnimmt, schneidet und dann an einer Anordnungsstation mit einer Lage des späteren Hygieneartikels verbindet. Eine derartige kinematisch aufwendige Konstruktion ist allerdings systembedingt möglicherweise fehleranfällig und schwierig und damit auch kostenintensiv zu warten. Des Weiteren ist durch die Kissengröße die Größe des elastischen Elements fest vorgegeben und kann nicht variabel und mehreren Produkten angepasst verändert werden. Eine Änderung der Produktgröße ist demnach nur möglich, wenn mindestens der komplette Kissensatz ausgetauscht wird. Zusätzlich führt das Schneiden der elastischen Elemente vor und hinter der Kissen dazu, dass die Enden des elastischen Elements nicht am Kissen fixiert sind und somit nicht in einem gedehnten Zustand gehalten werden können. Sie ziehen sich daher bereits vor dem Auftragen auf die Lage wieder zusammen, was zu einer unregelmäßigen Dehnung des elastischen Elements, insbesondere an deren Enden, beim fertigen Endprodukt führt.

Eine weitere Vorrichtung zum Herstellen von Hygieneartikeln mit einem elastischen Element ist aus der Druckschrift WO 2004/073568 A1 bekannt. Dort wird das elastische Material über eine Rollen- bzw. Scheibenkonstruktion geführt und später mit der Lage verklebt. Die Dehnung des elastischen Elements geschieht dabei während der Verklebung, durch eine Geschwindigkeitsdifferenz der Lagenbahn und des zugeführten elastischen Elements. Bei der Dehnung des elastischen Elements während dem Auftragen auf die Lagenbahn müssen die Rückstellkräfte bei der Dehnung somit von dem vorderen Ende des elastischen Elements gehalten werden. Da diese Kräfte bereits mit der ersten Verklebung des elastischen Elements auftreten ist es möglich, dass die bis dahin erfolgte Bindung des elastischen Elements zur Lagenbahn nicht ausreicht, um die Rückstellkräfte zu kompensieren. Daher ist es möglich, dass sich das bereits gedehnte elastische Element von der Lagenbahn löst oder tangential verschiebt, so dass die Dehnung vermindert wird oder ganz verloren geht.

Auch aus der Druckschrift DE 32 38 051 A1 ist ein Verfahren und eine Vorrichtung zum Aufbringen elastischer Elemente auf eine Materialbahn bekannt, bei der elastische Elemente während der Übergabe von einer Scheibe auf eine andere Scheibe gedehnt werden. Die elastischen Elemente werden in dieser Vorrichtung jeweils am Anfang und Ende festgehalten. Nachteilig an dieser Ausführung ist allerdings, dass die Scheiben zum Transport und zur Übergabe der elastischen Elemente über speziell angefertigte Formatvorlagen verfügen, weshalb ein Formatwechsel (beispielsweise im Rahmen eines Produktwechsels) einen Austausch und eine Neuanfertigung der Formatvorlagen bedingt. Dadurch entstehen lange Stillzeiten der Vorrichtung und zusätzliche Kosten bei einem Formatwechsel.

Die Druckschrift EP 1 162 162 A1 beschreibt ein Umladeverfahren und eine Umladevorrichtung zum Erhöhen oder Verringern der Umladegeschwindigkeit eines Werkstücks, wenn das Werkstück, dass sich auf einer Umladelinie bewegt zu einer anderen Umladelinie weitergereicht wird, Durch die vorgegebenen Größen der Aufnahmen für die Werkstücke ist eine Abänderung der Größen der Werkstücke vergleichsweise aufwendig.

In der US 2004/035521 A1 wird ebenfalls eine Vorrichtung beschrieben, die mit Hilfe von kreisförmig angeordneten Aufnahmekissen elastische, bereits zugeschnittene Elemente aufnimmt und sie dann über eine Verbindungsstation mit einer Lage des späteren Endprodukts verbindet. Auch diese Konstruktion ist möglicherweise fehleranfällig und kostenintensiv zu warten. Durch die vorgegebene Größe der Aufnahmekissen sind auch die Größen der elastischen Elemente vorgegeben und können nicht variiert werden. Eine Änderung der Produktgröße ist demnach nur möglich, wenn mindestens die komplette Aufnahmevorrichtung ausgetauscht wird.

Auch die Druckschrift DE 600 31 962 T2 offenbart ein Umladeverfahren und eine Umladevorrichtung bei der durch Veränderung der Auflade- bzw. Umladegeschwindigkeiten die Werkstücke weitertransportiert werden. Nachteilig ist auch hier, dass die Größen der Aufnahmevorrichtungen nicht ohne weiteres verändert werden können.

Aus der EP 0 869 755 B1 ist eine Vorrichtung und ein Verfahren bekannt, bei dem verlängerte elastische Teile auf eine sich fortlaufend bewegende Bahn aus ebenfalls verlängertem und elastischem Material aufgebracht wird. Da bei dieser Vorrichtung und diesem Verfahren die aufzubringenden elastischen Teile sowie auch die elastische Materialbahn verlängert werden besteht die Gefahr, dass die Dehnungen verloren gehen bzw. nicht zuverlässig miteinander verbunden werden. Der Erfindung liegt daher die Aufgabe zu Grunde, ein Elastik-Applikator und ein Verfahren zum Betrieb eines Elastik-Applikators der oben genannten Art anzugeben, der auf einfachste Weise ein zuverlässiges und gleichmäßiges Aufbringen eines elastischen Elements auf eine Lagenbahn ermöglicht und gleichzeitig besonders einfach und insbesondere ohne aufwändige Umbauten elastische Elemente mit unterschiedlichen Größen bzw. Längen und Dehnungskoeffizienten aufbringen kann.

Bezüglich des Elastik-Applikators wird diese Aufgabe erfindungsgemäß gelöst mit den Merkmalen des Anspruchs 1.

Bezüglich des Verfahrens wird die genannte Aufgabe gelöst, indem das elastische Element zunächst durch ein Scheibenpaar geführt wird bevor es mit der Materiallage verbunden wird, wobei das elastische Element durch das Scheibenpaar gedehnt wird, indem die im Führungsweg des elastischen Elements liegende erste Scheibe des Scheibenpaares dem Betrage nach mit einer geringeren Geschwindigkeit dreht als die im Führungsweg des elastischen Elements liegende zweite Scheibe des Scheibenpaares und, dass die Dreh- bzw. Umfangsgeschwindigkeit der zweiten Scheibe und die Geschwindigkeit der zugeführten Materiallage dem Betrage nach im Wesentlichen gleich sind und das elastische Material an den Scheiben durch eine Anzahl von mantelseitig umlaufenden Saugöffnungen gehalten wird.

Die Erfindung geht dabei von der Überlegung aus, dass eine vollständige und gleichmäßige Aufbringung des gedehnten elastischen Elements auf die Materiallage, beispielsweise eine Vliesstofflage, dann erreicht werden kann, wenn das elastische Element bei der Aufbringung bzw. Verklebung auf die Materiallage nicht zusätzlich gedehnt wird oder werden muss. Dies kann erreicht werden, indem auf eine gewollte Relativgeschwindigkeit des elastischen Elements und der zugeführten Materiallage bei der Verklebung verzichtet wird. Ein Aufbringen eines gedehnten elastischen Elements auf eine Materiallage bei gleicher Geschwindigkeit wird ermöglicht, wenn das elastische Element bereits vor der Aufbringung bzw. Verklebung auf die Materiallage in die gewünschte Länge bzw. mit dem gewünschten Stretchfaktor gedehnt wurde. Eine derartige Dehnung des elastischen Elements vor der eigentlichen Applikation auf die Materialbahn soll daher mit Hilfe zweier vorgelagerter Scheiben erreicht werden, die gemeinsam ein Scheibenpaar bilden. Dazu kann die Rotationsgeschwindigkeit der beiden Scheiben derart eingestellt werden, dass das elastische Element beim Übergang von einer Scheibe zur nächsten den gewünschten Stretchfaktor bzw. die gewünschte Dehnung erhält.

Weiterhin geht die Erfindung von der Überlegung aus, dass eine möglichst flexible Einstellung verschiedener Dehnungsparameter aber auch Längen des elastischen Materials und somit auch verschiedener Produktgrößen des Fertigproduktes dann erreicht werden kann, wenn auf den Austausch von speziell für eine Formatlänge und -dehnung ausgelegte Transport- und/oder Befestigungseinheiten für das elastische Element verzichtet werden kann. Dazu ist zumindest eine Scheibe des Scheibenpaares, bevorzugt aber beide Scheiben des Scheibenpaares, mit mantelseitig umlaufenden Saugöffnungen versehen.

Um die Relativgeschwindigkeit der beiden Scheiben zur Dehnung des elastischen Elements dabei leicht an den gewünschten Stretchfaktor anpassen zu können, sind die Antriebseinheiten in bevorzugter Ausführung als Direktantriebe ausgebildet, also als stufenlos und direkt einstellbare Antriebseinheiten. Somit können ohne Verzögerungen verschiedene Produkte mit unterschiedlichen Strechfaktoren nacheinander hergestellt werden und auch der Stretchfaktor entlang eines einzelnen elastischen Elements verändert werden. Für übliche Stretchfaktoren im Bereich von 60 % bis 100 % sind beispielsweise Geschwindigkeitsverhältnisse von etwa 1.6 bis 2 optimal. Da die Scheiben vorteilhafterweise mit einer möglichst großen Anzahl von Saugöffnungen ausgestattet sind, die mantelseitig umlaufend sind, kann das elastische Element in jedem Bereich zwischen zwei Öffnungen während der Übergabe abschnittsweise gedehnt werden. Üblicherweise überdeckt ein ungedehnter elastischer Streifen auf einer Scheibe je nach Länge 10 bis 25 Saugöffnungen, ein gedehnter Streifen auf einer Scheibe bei gleicher Geometrie der Scheiben entsprechend der Dehnung mehr. Eine große Anzahl an Saugöffnungen auf den Scheiben führt zu einem geringen Abstand auf der Mantelfläche zwischen den Saugöffnungen. Dadurch bleibt, unabhängig von der Länge der elastischen Elemente und unabhängig vom Stretchfaktor, nur ein sehr kleines Stück am Anfang und Ende des Streifens ungedehnt, nämlich der Teil der vor der ersten überdeckten Saugöffnung und nach der letzten überdeckten Saugöffnung auf der letzten Scheibe des Scheibenpaares zum Liegen kommt. Dieser Randbereich beträgt üblicherweise 1-2 mm und kann somit bei entsprechender Anzahl von Saugöffnungen besonders klein gehalten werden.

Zur Vermeidung einer tangentialen Verschiebung des elastischen Elements beim Übergang zwischen den Scheiben, ist die Oberfläche der Rollen in vorzugsweiser Ausführung mit einer rauen Beschichtung versehen. Die Rauhigkeit der Oberfläche ist dabei gezielt dazu ausgelegt, die tangentiale Kraft auf die elastischen Elemente auszugleichen, damit ein Verrutschen der elastischen Elemente vermieden werden kann.

Sowohl der Halt des elastischen Elements als auch die Übergabe von einer Scheibe auf die nächste wird in bevorzugter Ausführung über eine Ansaugkraft erreicht, die durch eine Unterdruckeinheit erzeugt wird und durch die Saugöffnungen auf das elastische Element wirkt.

Für den sicheren Transport der elastischen Elemente entlang der Scheiben bzw. Walzen sind die Unterdruckeinheiten in besonders vorteilhafter Ausführung ortsfest ausgebildet und erstrecken sich über den gesamten Bereich der Scheiben, entlang dessen das elastische Element transportiert wird. Durch die ortsfeste Anordnung der Unterdruckeinheit dreht sich die Mantelfläche mit den umlaufenden Saugöffnungen stetig über die Unterdruckeinheit hinweg, sodass zu jedem Zeitpunkt eine ausreichende Anzahl von Saugöffnungen mit entsprechendem Unterdruck zur Aufnahme des elastischen Elements bereit steht, unabhängig von der Länge des elastischen Materials.

In vorteilhafter Ausgestaltung verfügt die Unterdruckeinheit über eine Anzahl von Kammern, in denen der Unterdruck, beispielsweise erzeugt durch eine Vakuumpumpe, variiert werden kann. Dabei lässt sich der Unterdruck beispielsweise derart steuern, dass bei der Übergabe des elastischen Elements ein für die gleichmäßige Übergabe optimierter Druck anliegt.

Das elastische Element kann besonders zuverlässig auf den gewünschten Stretch- bzw. Dehnungsfaktor gedehnt werden, wenn der Zuschnitt der elastischen Elemente vor der Dehnung geschieht. Dazu wird in vorteilhafter Ausgestaltung das elastische Element vor der Übergabe auf das Scheibenpaar mittels einer Schneidevorrichtung auf die gewünschte Länge geschnitten. Eine Beeinflussung des Stretch- bzw. Dehnungsfaktors durch den Schneidevorgang entfällt somit.

Die Schneidevorrichtung ist dazu in vorteilhafter Ausgestaltung vor den Rollen angeordnet und schneidet die elastischen Elemente gegen eine Leiste mit glatter Oberfläche. Damit ein Zurückflitschen des elastischen Elements beim Schneidevorgang vermieden wird, wird in besonders vorteilhafter Ausgestaltung nicht gegen eine feste Leiste geschnitten, sondern gegen die erste Überführungsscheibe mit glatter Oberfläche. Dies führt dazu, dass eine in der Überführungscheibe befindliche Unterdruckeinheit das geschnittene elastische Element und die Materialbahn des elastischen Elements an die Rolle ansaugt und somit vor, während und nach dem Schneidevorgang in Position hält.

In vorteilhafter Ausführung wird das gedehnte elastische Element anschließend mit einer Materiallage, beispielsweise bestehend aus einem Vliesstoff, verbunden. Dabei kann die Materiallage vor dem Kontakt mit dem elastischen Element durch eine Klebeeinheit für die Applikation, beispielsweise durch Auftragung einer Klebeschicht auf die Materiallage, entsprechend vorbereitet werden. Alternativ ist auch die Verbindung des elastischen Elementes und der Materiallage durch eine Verprägung beider möglich.

Die mit der Erfindung erzielten Vorteile bestehen insbesondere darin, dass bei der Verbindung des elastischen Materials und der Lagenbahn bei gleicher Geschwindigkeit und somit fehlender Relativgeschwindigkeit zusätzliche Zugkräfte auf die Materialien vermieden werden können. Dadurch ist eine besonders gleichmäßige Dehnung und Aufbringung des elastischen Elements möglich. Zusätzlich wird durch die vorgeschaltete Dehnung des elastischen Elements mittels zweier Scheiben oder auch Rollen mit unterschiedlicher Geschwindigkeit auf einfachste Weise erreicht, dass die gewünscht Dehnung durch Variation der Drehgeschwindigkeiten variabel und kurzfristig den Bedürfnissen entsprechend angepasst werden kann. Auch die weiter vorgeschaltete Schnittvorrichtung erlaubt einen individuell und jederzeit veränderbaren Zuschnitt des elastischen Elements. Durch die Anordnung der Saugöffnungen können beliebig lange elastische Elemente sowohl bei der Übergabe von der einen Scheibe auf die nächste Scheibe gedehnt werden als auch beliebig lange elastische Elemente von diesen Scheiben gehalten werden. Ein zeit-, arbeit- und kostenintensiver Austausch von Befestigungseinheiten, die auch zum kurzzeitigen Stillstand der Produktionsabläufe führen würden, kann somit vermieden werden.

Ein Ausführungsbeispiel wird anhand einer Zeichnung näher erläutert. Darin zeigen:
- FIG. 1: ein Elastik-Applikator mit einer Schneidevorrichtung gegen eine Leiste,
- FIG. 2: ein Elastik-Applikator mit einer Schneidevorrichtung gegen eine glatte Scheibe,
- FIG. 3: eine Scheibe mit mantelseitig umlaufenden Saugöffnungen,
- FIG. 4: Unterdrucksystem eines Elastik-Applikators,
- FIG. 5: Verfahren zur Dehnung eines elastischen Elements.

Gleiche Teile sind in allen Figuren mit denselben Bezugszeichen versehen.

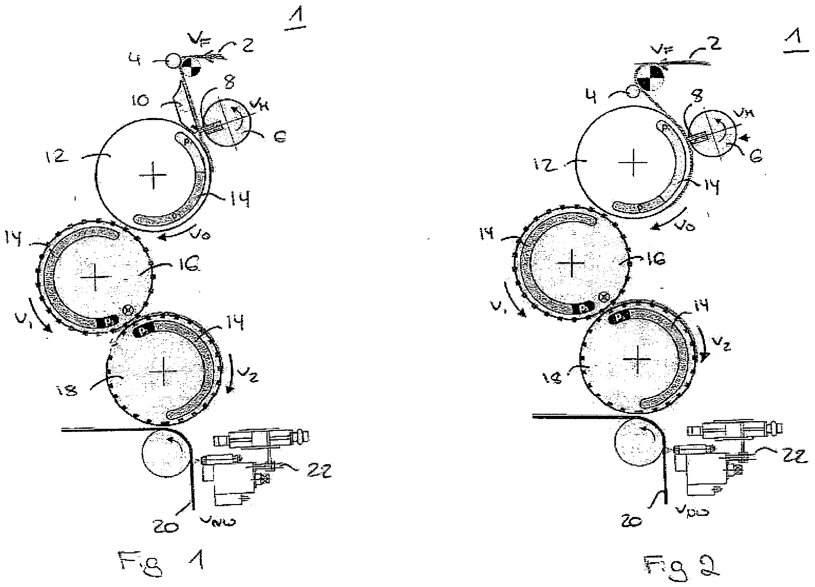

Dem Elastik-Applikator 1 gemäß FIG. 1 wird aus einer externen und nicht dargestellten Zuführeinheit eine Materialbahn 2 eines elastischen Elements zugeführt. Dieses elastische Element könnte beispielsweise aus Schaumstoff oder einem anderen Material mit den gewünschten elastischen Eigenschaften bestehen. Die Materialbahn 2 wird über Umlenkrollen 4 zu einer Schneidevorrichtung 6 des Elastik-Applikators 1 geführt. Die Schneidevorrichtung 6 umfasst eine drehbare Scheibe, an der eine Schneidekante bzw. ein Schneidmesser 8 befestigt ist. Als Schneidfläche ist bei der Schneidevorrichtung 6 im vorliegenden Ausführungsbeispiel eine feste Gegenleiste 10 vorgesehen, die über eine glatte Oberfläche verfügt. Die Materialbahn 2 wird entlang dieser Gegenleiste 10 geführt und durch das Schneidmesser 8 in die gewünschte Größe des elastischen Elements geschnitten. Dabei kann durch Variation der Drehgeschwindigkeit der Scheibe v_{M}, an der das Schneidmesser 8 befestigt ist, in Relation zur Geschwindigkeit der Materialbahn des elastischen Elements v_{F} die gewünschte Größe der elastischen Elemente produktindividuell eingestellt werden.

Das zugeschnittene elastische Element wird dann einer Überführungsscheibe bzw. -walze 12 übergeben, auf der das elastische Element mittels eines durch einer Anzahl von Unterdruckeinheiten 14 erzeugten Unterdrucks auf der Überführungsscheibe 12 gehalten wird. Die Unterdruckeinheiten 14 sind ortsfest neben oder in der Scheibe angeordnet und drehen sich nicht mit dieser mit. Zur Übertragung des Unterdrucks auf das elastische Element sind an der Oberfläche der Überführungsscheibe 12 eine Vielzahl von Saugöffnungsreihen 24 vorgesehen, die in Form von Saugbohrungen ausgeführt sind. Die Überführungsscheibe 12 dreht sich mit einer einstellbaren Geschwindigkeit v₀, die größer oder gleich der Geschwindigkeit des elastischen Elements v_{F} sein kann.

Die Überführungsscheibe 12 übergibt dann das elastische Element einem Scheiben- bzw. Walzenpaar 16, 18. Auch dieses Scheibenpaar 16, 18 verfügt wie die Überführungsscheibe 12 über eigene Unterdruckeinheiten 14, die ortsfest neben oder in den einzelnen Scheiben angeordnet sind. Diese Unterdruckeinheiten 14 erzeugen ein einstellbares Druckprofil, dass zum einen das elastische Element besonders zuverlässig auf den Scheiben hält und zum anderen einen gleichmäßigen und rutschfreien Übergang des elastischen Elements von Scheibe 16 auf die Scheibe 18 ermöglicht.

Die erste Scheibe des Scheibenpaares 16 dreht mit einer einstellbaren Geschwindigkeit v₁. Diese Geschwindigkeit kann der Geschwindigkeit v₀ der Überführungsscheibe 12 entsprechen, um einen möglichst gleichmäßigen Übergang des elastischen Materials zu ermöglichen. Es ist aber auch möglich, die erste Scheibe des Scheibenpaares 16 mit einer höheren Geschwindigkeit drehen zu lassen, damit der Auszug, d. h. der Abstand zwischen zwei geschnittenen elastischen Elementen vergrößert wird. Dies kann individuell und den Anforderungen entsprechend eingestellt werden. Bei der Übergabe des elastischen Elements von der Überführungsscheibe 12 auf die Scheibe 16 wird das elastische Element vorteilhafterweise nicht bzw. nur sehr geringfügig gedehnt, auch wenn die Geschwindigkeit v₁ > v₀ ist. Dies wird erreicht zum einen durch sehr geringen Unterdruck am Ende der Saugstrecke der Überführungsscheibe 12, sowie eine glatte Oberfläche der Überführungsscheibe 12. Somit können die geschnittenen Streifen von der Überführungsscheibe gezogen werden, ohne dass eine nennenswerte Dehnung der Streifen stattfindet, d.h. der Geschwindigkeitsunterschied der Scheiben 12 und 16 führt vollständig dazu, dass ein Abstand zwischen den Streifen erzeugt wird.

Die zweite Scheibe des Scheibenpaares 18 dreht mit einer einstellbaren Geschwindigkeit v₂. Diese Geschwindigkeit ist höher als die Geschwindigkeit der ersten Scheibe des Scheibenpaares v₁. Durch die Geschwindigkeitsdifferenz zwischen der ersten Scheibe und der zweiten Scheibe des Scheibenpaares 16, 18 wird das elastische Element bei der Übergabe gedehnt. Der Dehnungskoeffizient bzw. Stretchfaktor hängt dabei direkt von der Geschwindigkeitsdifferenz ab. Übliche Dehnungskoeffizienten von etwa 60 % bis 100 % können beispielsweise bei gleichen Scheibendurchmessern bei Geschwindigkeitsverhältnissen im Bereich von v₂ = 1.6 - 2 v₁ erreicht werden.

Dabei ist es vorteilhaft, wenn sich die Scheiben zusätzlich in Takt drehen, das bedeutet, dass sich die Scheiben pro Maschinentakt, d.h. pro hergestelltes Produkt, immer um eine je nach Produkt einzustellende ganzzahlige Anzahl von Ansaugöffnungen 24 weiterdrehen. Dadurch können Schwankungen in der Position sowie der Länge der gedehnten Streifen verringert werden.

Die Oberfläche der beiden Scheiben des Scheibenpaars 16, 18 kann rau ausgeführt werden, um eine zusätzliche tangentiale Verschiebung und Verminderung des Stretchfaktors des gedehnten elastischen Elements zu unterdrücken. Die Rauhigkeit der Oberfläche dient somit der Reibwerterhöhung zwischen dem elastischen Element und der Scheibe und kann an das Material angepasst werden. Bei schaumstoffartigen Materialen für das elastische Element ist beispielsweise eine relative grobe Körnung denkbar, bei anderen Materialien kann diese unter Umständen verringert werden oder ganz auf die raue Oberfläche verzichtet werden.

Die Druckprofile der Unterdruckkammern 14 sind im Ausführungsbeispiel nach FIG. 1 so gewählt, dass der größte Unterdruck und damit die größte Ansaugkraft beim Dehnungsvorgang zwischen den beiden Scheiben des Scheibenpaares 16, 18 existiert. Bei den vorherigen Übergängen auf die Überführungsscheibe 12 und auf die erste Scheibe des Scheibenpaares 16 ist ein geringerer Unterdruck eingestellt, damit eine Übergabe bzw. ein Runterziehen des elastischen Elements durch die Scheiben erleichtert wird. Im Ausführungsbeispiel nach FIG. 1 sind dazu beispielhaft drei Druckbereiche angegeben. Diese können aber im Rahmen der obigen Anforderungen an die Betriebsparameter, wie beispielsweise die Geschwindigkeiten der Scheiben oder auch an das Material des elastischen Elements angepasst werden. Es ist also durchaus ein anderes als im Ausführungsbeispiel nach FIG. 1 dargestelltes Druckprofil denkbar.

Die zweite Scheibe des Scheibenpaares 18 trägt anschließend das gedehnte elastische Element auf eine zugeführte Materialbahn 20, beispielsweise einen Vliesstoff, auf. Dazu kann die Materialbahn 20 zuvor mit Hilfe einer Klebeeinheit 22 zur Applikation mit dem elastischen Element vorbereitet werden. Für eine besonders sichere und gleichmäßige Auftragung des gedehnten elastischen Elements auf die Materialbahn 20 sind die Geschwindigkeiten der zweiten Scheibe des Scheibenpaares v₂ und der Materialbahn v_{NW} dem Betrage nach im Wesentlichen gleich. Es findet somit keine weitere zusätzliche Dehnung des elastischen Elements beim Auftragen auf die Materialbahn 20 statt. Somit können zusätzliche Zugkräfte, die bei der Dehnung des elastischen Elements während dem Verkleben auftreten, vermieden werden. Alternativ zum dargestellten Verkleben des gedehnten elastischen Elements mit der Materialbahn ist auch Verprägen beider möglich.

Die Saugöffnungen 24, durch die der Unterdruck auf das elastische Element übertragen wird und an den Scheiben 12, 16, 18 gehalten wird sind dabei über die komplette Mantelfläche verteilt. Dadurch ist es möglich beliebig lange elastische Elemente zu transportieren und zu halten.

Das Ausführungsbeispiel nach den FIG. 2 unterscheiden sich von dem aus FIG. 1 im Wesentlichen durch die Anordnung und Funktionsweise der Schneidevorrichtung 6. Im Ausführungsbeispiel nach FIG. 2 wird die Materialbahn des elastischen Elements direkt an die Überführungsscheibe 12 übergeben und die Scheidevorrichtung schneidet die Materialbahn auf der glatten Überführungsscheibe 12. Das elastische Element wird somit bereits beim Schneidevorgang von der Unterdruckeinheit 14 fest an der Scheibe gehalten. Ein Zurückflitschen des elastischen Elements beim Schneidevorgang wird somit unterdrückt.

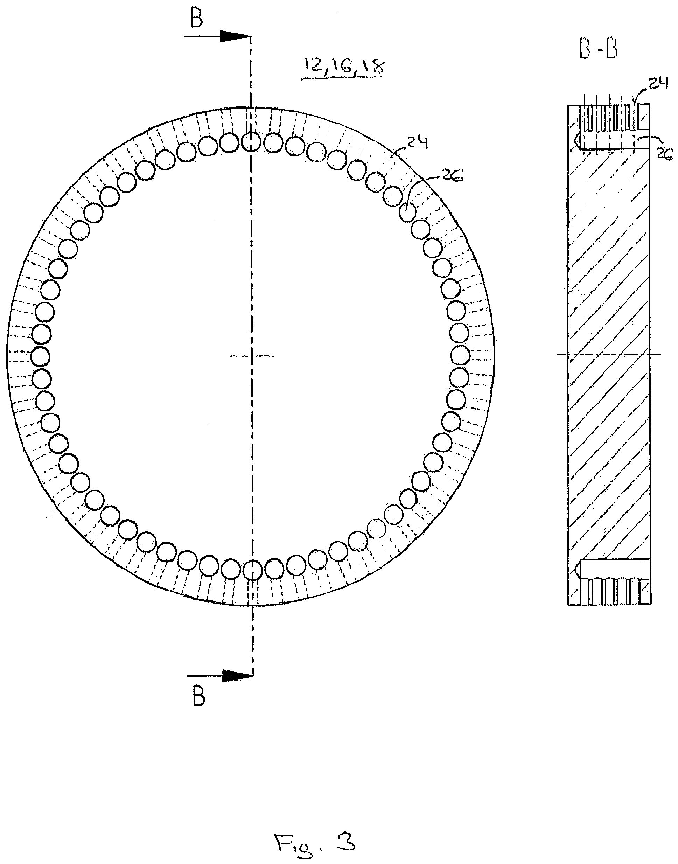

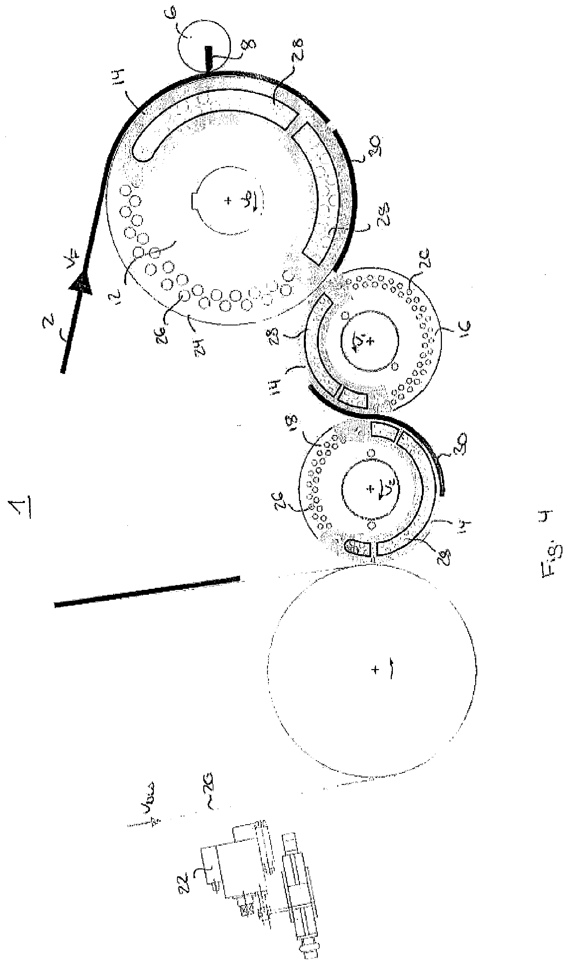

Eine Scheibe 12, 16, 18 mit einer Anzahl von mantelseitig umlaufenden Saugöffnungen 24 ist in Fig. 3 dargestellt. An der dargestellten Stirnseite der Scheibe 12, 16, 18 sind eine Anzahl von Öffnungen zu Saugkanälen 26 dargestellt, die jeweils mit einer Reihe von Saugöffnungen 24 zusammenwirken, die als Saugbohrungen ausgebildet sind. Sobald die Saugkanäle 26 bei Drehung der Scheibe 12, 16, 18 die Unterdruckeinheiten 14 passieren liegt an den Saugöffnungen auf der Mantelfäche der Scheibe 12, 16, 18 der entsprechende Unterdruck an, so dass das elastische Material sicher auf der Scheibe 12, 16, 18 gehalten werden kann. Im vorliegenden Ausführungsbeispiel ist dabei nur ein Ring von Saugkanälen 26 dargestellt, es aber auch möglich, dass die Saugkanäle 26 in mehreren Ringen versetzt zueinander angeordnet sind, wie dies beispielsweise für zwei Ringe in der Scheibenanordnung des Ausführungsbeispiels nach Fig. 4 dargestellt wird.

Ein beispielhaftes Unterdrucksystem ist im Ausführungsbeispiel nach Fig. 4 dargestellt. Jede Unterdruckeinheit 14 umfasst dabei mehrere Unterdruckkammern 28, die getrennt voneinander angesteuert werden können und individuell mit Unterdruck beaufschlagt werden können. Diese Unterdruckkammern 28 sind dabei derart an den Scheiben 12, 16, 18 angeordnet, dass die Schreiben 12, 16, 18 mit den Saugkanälen 26 an den Unterdruckkammern 28 vorbeigeführt werden. Dabei ist die Anzahl, Ausführung und Positionierung der dargestellten Unterdruckkammern 28 nur beispielhaft anzusehen, insbesondere können weitere Unterdruckkammern 28 vorgesehen sein, um ein optimiertes Unterdruckprofil bereitstellen zu können. Dazu umfasst jede Unterdruckkammer 28 eigene Zuleitungen.

Durch die Möglichkeit der individuellen Ansteuerung der Unterdruckkammern 28 wird ein optimaler Transport und eine optimale Dehnung der elastischen Elemente 30 erst ermöglicht. So ist es beispielsweise denkbar, die in Transportrichtung des elastischen Elements 30 letzte Unterdruckkammer 28 der Überführungsscheibe 12 nur mit einem geringe Unterdruck, insbesondere im Vergleich zur Unterdruckkammer 28 der Scheibe 16, die das elastische Element von der Überführungsscheibe 12 aufnimmt, beaufschlagt wird. In Kombination mit einer glatten Oberfläche der Überführungsscheibe 12 wird dadurch erreicht, dass die geschnittenen Streifen des elastischen Elements 30 von der Überführungsscheibe gezogen werden, ohne dass eine nennenswerte Dehnung der Streifen stattfindet, d.h. der Geschwindigkeitsunterschied der Scheiben 12 und 16 führt vollständig dazu, dass ein Abstand zwischen den Streifen erzeugt wird. Somit ist also möglich die Abstände der einzelnen elastischen Elemente 30 zu steuern.

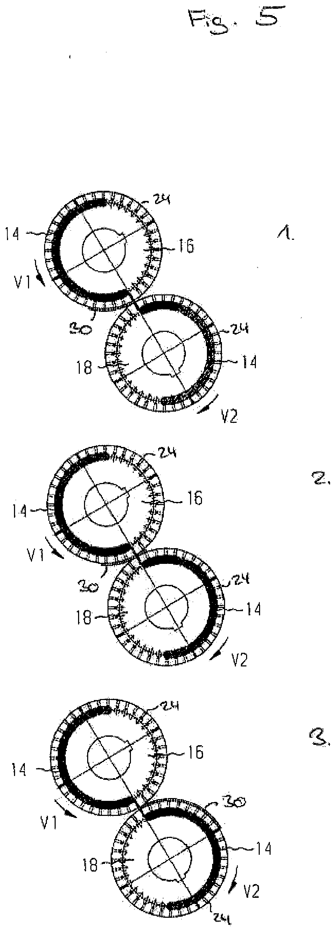

Das Verfahren zur Dehnung des elastischen Elements ist in Fig. 5 exemplarisch anhand von drei skizzierten Verfahrensschritten dargestellt. Das elastische Element 30 wird dabei im ersten Verfahrensschritt durch den Unterdruck an den Saugöffnungen 24 der ersten Scheibe des Scheibenpaars 16 gehalten. Das elastische Element 30 ist dabei derart auf der Scheibe 16 positioniert, dass die Vorderkante an einer Reihe von Saugöffnungen 24 liegt. Im zweiten Verfahrensschritt wird diese Vorderkante des elastischen Elements durch eine Reihe von Saugöffnungen 24 der zweiten Scheibe des Scheibenpaares 18 aufgenommen. Die Reihe an Saugöffnungen 24 der ersten Scheibe 16 hat dabei ihre zugehörige Unterdruckkammer 28 verlassen, weshalb die Vorderkante des elastischen Elements 32 nicht mehr von der ersten Scheibe 16 gehalten werden kann. Das elastische Element 30 wird anschließend Saugöffnungsreihe für Saugöffnungsreihe an die zweite Scheibe 18 übergeben. Da sich diese zweite Scheibe 18 allerdings mit einer höheren Umfangsgeschwindigkeit v₂ > v₁ als die erste Scheibe 16 dreht, wird das elastische Element 30 bei der Übergabe zusätzlich gedehnt und liegt auf der zweiten Scheibe 18 im gedehnten Zustand, wie dies im dritten Verfahrensschritt dargestellt ist.

Mit allen Ausführungsbeispielen ist es somit möglich über die Einstellung der Geschwindigkeiten des Scheibenpaares v₁ und v₂ stufenlos einen gewünschten Stretchfaktor des elastischen Materials zu erzielen. Dies ist auch während des Betriebes des Elastik-Applikators jederzeit möglich. Damit werden zusätzliche Ruhezeiten und aufwendige Umrüstungen der Anlage bei Produktwechsel bzw. Formatwechsel vermieden. Weiterhin kann durch die direkt vorgelagerte Schneideeinheit auf Formatteile beim Zuschnitt verzichtet werden und somit jegliche Länge stufenlos eingestellt werden. Durch die ausschließlich einfachen, rotierenden Bewegungen der einzelnen Komponenten der Anlage und die Vermeidung von komplexen Porduktionsabläufen können die Fehleranfälligkeit der Anlage und Wartungs- und Reinigungszeiten gering gehalten werden.

## Patentansprüche

1. Elastik-Applikator (1) zum Aufbringen eines elastischen Elements auf eine Materiallage , mit einem Scheibenpaar (16, 18) und jeweils einer Antriebseinheit zum Antreiben der beiden Scheiben des Scheibenpaares (16, 18), wobei die Scheiben des Scheibenpaares (16, 18) und die Antriebseinheiten zum Betrieb mit voneinander dem Betrage nach verschiedenen Geschwindigkeiten (v1, v2) ausgelegt sind, wobei zumindest eine Scheibe des Scheibenpaares (16,18) mantelseitig umlaufend eine Anzahl von Saugöffnungen (24) umfasst, und wobei die beiden Scheiben des Scheibenpaares (16, 18) über jeweils eine Unterdruckeinheit (14) verfügen, **dadurch gekennzeichnet, dass** die Unterdruckeinheit (14) jeweils mehrere Unterdruckkammern (28) umfasst, die getrennt voneinander ansteuerbar und individuell mit Unterdruck beaufschlagbar sind.

2. Elastik-Applikator (1) nach Anspruch 1, **dadurch gekennzeichnet, dass** die Antriebseinheiten derart ausgebildet sind, dass die Geschwindigkeiten (v₁, v₂) stufenlos und direkt einstellbar bzw. veränderbar sind.

3. Elastik-Applikator (1) nach Anspruch 1 oder 2, **dadurch gekennzeichnet**, das die beiden Scheiben des Scheibenpaares (16, 18) mit einer rauen Oberflächenbeschichtung versehen sind.

4. Elastik-Applikator (1) nach einem der Ansprüche 1 bis 3, **dadurch gekennzeichnet, dass** die Unterdruckeinheit (14) ortsfest ausgebildet ist und sich im Wesentlichen entlang des gesamten Transportweges des elastischen Elements erstreckt.

5. Elastik-Applikator (1) nach einem der Ansprüche 1 bis 4, **dadurch gekennzeichnet, dass** eine Schneidevorrichtung (6) vorgesehen ist.

6. Elastik-Applikator (1) nach Anspruch 5, **dadurch gekennzeichnet, dass** die Schneidevorrichtung (6) ein Schneidmesser (8) umfasst und als Schneidfläche eine planare Gegenleiste (10) vorgesehen ist.

7. Elastik-Applikator (1) nach Anspruch 5, **dadurch gekennzeichnet, dass** die Schneidevorrichtung (6) mindestens ein Schneidmesser (8) umfasst und als Schneidfläche eine zusätzliche Scheibe (12) vorgesehen ist.

8. Verfahren zum Betreiben eines Elastik-Applikators (1) zum Aufbringen eines elastischen Elements auf eine Materiallage (20) nach einem der Ansprüche 1 bis 7, bei dem das elastische Element zunächst durch ein Scheibenpaar (16, 18) geführt wird bevor es mit der Materiallage (20) verbunden wird, wobei das elastische Element durch das Scheibenpaar (16, 18) gedehnt wird, indem die im Führungsweg des elastischen Elements liegende erste Scheibe des Scheibenpaares (16) dem Betrage nach mit einer geringeren Geschwindigkeit (v₁) dreht als die im Führungsweg des elastischen Elements liegende zweite Scheibe des Scheibenpaares (18) und, dass die Drehgeschwindigkeit der zweiten Scheibe (v₂) und die Geschwindigkeit der zugeführten Materiallage (v_{NW}) dem Betrage nach im Wesentlichen gleich sind und wobei das elastische Material an den Scheiben durch eine Anzahl von mantelseitig umlaufenden Saugöffnungen gehalten wird.

## Claims

1. Elastic applicator (1) for applying an elastic element to a material layer, comprising a pair of discs (16, 18) and a driving unit in each case for driving the two discs of the pair of discs (16, 18), wherein the discs of the pair of discs (16, 18) and the driving units are designed to operate with different speed values (v1, v2), wherein at least one disc of the pair of discs (16, 18) comprises on the circumferential outer side a number of suction openings (24), and wherein the two discs of the pair of discs (16, 18) are each provided with a vacuum unit (14), **characterised in that** each vacuum unit (14) comprises a plurality of vacuum chambers (28), which may be independently controlled and individually subjected to a vacuum.

2. Elastic applicator (1) according to claim 1, **characterised in that** the driving units are designed in such a way that the speeds (v₁, v₂) may be adjusted or modified in a continuous and direct way.

3. Elastic applicator (1) according to either claim 1 or claim 2, **characterised in that** the two discs of the pair of discs (16, 18) are provided with a rough surface coating.

4. Elastic applicator (1) according to any of claims 1 to 3, **characterised in that** the vacuum unit (14) is stationary and extends substantially along the entire transport path of the elastic element.

5. Elastic applicator (1) according to any of claims 1 to 4, **characterised in that** a cutting device (6) is provided.

6. Elastic applicator (1) according to claim 5, **characterised in that** the cutting device (6) comprises a cutting knife (8) and the cutting surface is formed by a planar counter ledge (10).

7. Elastic applicator (1) according to claim 5, **characterised in that** the cutting device (6) comprises at least one cutting knife (8) and the cutting surface is formed by an additional disc (12).

8. Method for operating an elastic applicator (1) for applying an elastic element to a material layer (20) according to any of claims 1 to 7, in which the elastic element is initially guided by a pair of discs (16, 18), before it is connected to the material layer (20), wherein the elastic element is expanded by the pair of discs (16, 18), in which the first disc of the pair of discs (16), which is positioned in the guiding path of the elastic element, rotates with a lower speed value (v₁) than the second disc of the pair of discs (18) positioned in the guiding path of the elastic element and that the rotational speed of the second disc (v₂) and the speed of the conveyed material layer (v_{NW}) have substantially equal values and wherein the elastic material is held on the discs by a number of suction openings, which are circumferentially disposed on the outer side.

## Revendications

1. Applicateur élastique (1) destiné à appliquer un élément élastique sur une couche de matériau, à l'aide d'une paire de disques (16, 18) et respectivement une unité d'entraînement pour entraîner les deux disques de la paire de disques (16, 18), où les disques de la paire de disques (16, 18) et les unités d'entraînement sont conçues pour fonctionner avec des vitesses (v₁, v₂) de valeurs différentes, au moins un disque de la paire de disques (16, 18) comprenant un certain nombre d'ouvertures d'aspiration (24) sur sa périphérie, et les deux disques de la paire de disques (16, 18) disposant respectivement d'une unité de pression négative (14), **caractérisé en ce que** l'unité de pression négative (14) comprend respectivement plusieurs chambres de pression négative (28) pouvant être activées séparément les unes des autres et pouvant être sollicitées individuellement par pression négative.

2. Applicateur élastique (1) selon la revendication 1, **caractérisé en ce que** les unités d'entraînement sont conçues de telle sorte que les vitesses (v₁, v₂) puissent être réglées ou resp. modifiées en continu et directement.

3. Applicateur élastique (1) selon la revendication 1 ou 2, **caractérisé en ce que** les deux disques de la paire de disques (16, 18) sont munis d'un revêtement de surface rugueux.

4. Applicateur élastique (1) selon une des revendications 1 à 3, **caractérisé en ce que** l'unité de pression négative (14) est conçue fixe et s'étend pour l'essentiel le long de la totalité du parcours de l'élément élastique.

5. Applicateur élastique (1) selon une des revendications 1 à 4, **caractérisé en ce qu'**il est prévu un dispositif de découpe (6).

6. Applicateur élastique (1) selon la revendication 5, **caractérisé en ce que** le dispositif de découpe (6) comprend une lame de découpe (8) et est muni d'une contre-lame plane (10) comme surface de découpe.

7. Applicateur élastique (1) selon la revendication 5, **caractérisé en ce que** le dispositif de découpe (6) comprend au moins une lame de découpe (8) et est muni d'un disque supplémentaire (12) comme surface de découpe.

8. Procédé de fonctionnement d'un applicateur élastique (1) pour appliquer un élément élastique sur une couche de matériau (20) selon une des revendications 1 à 7, dans lequel l'élément élastique est d'abord conduit à travers une paire de disques (16, 18) avant d'être lié à la couche de matériau (20), l'élément élastique étant étendu à travers la paire de disques (16, 18), où le premier disque de la paire de disques (16) disposé sur le parcours de l'élément élastique tourne avec une vitesse (v₁) plus faible que celle du second disque (v₂) de la paire de disques (18) disposé sur le parcours de l'élément élastique, et en ce que la vitesse de rotation du second disque (v₂) et la vitesse de la couche de matériau introduite (v_{NW}) sont de valeurs sensiblement identiques, et où le matériau élastique est maintenu sur le disque par un certain nombre d'ouvertures d'aspiration sur sa périphérie.
